Europäisches Patentamt

European Patent Office

Office européen des brevets

(19)

(11) Publication number: **0 124 732**
**A2**

(12) **EUROPEAN PATENT APPLICATION**

(21) Appplication number: **84103161.0**

(22) Date of filing: **22.03.84**

(51) Int. Cl.³: **A 61 F 15/00**
**A 61 F 13/02**

(30) Priority: **09.05.83 US 492685**

(43) Date of publication of application:
**14.11.84 Bulletin 84/46**

(84) Designated Contracting States:
**AT BE CH DE FR GB IT LI LU NL SE**

(71) Applicant: **Norwood Industries, Inc.**
**100 North Morehall Road**
**Malvern Pennsylvania 19355(US)**

(72) Inventor: **Karakas, Arbort Frederick**
**Apartment L3 1100 West Chester Pike**
**West Chester Pennsylvania 19380(US)**

(74) Representative: **Mayer, Hans Benno**
**Via dell'Orso 7/A**
**I-20121 Milano(IT)**

(54) Packaging system for an occlusive dressing.

(57) Occlusive dressings are packaged in dressing units by continuously transporting dressing constructs to apparatus including a cutting means where the release liner, adhesive and polymeric film forming a composite on a first carrier sheet are cut through, the excess composite is removed to expose the margin of the first carrier sheet, a second carrier sheet is superimposed on the composite and the carrier sheets are sealed to each other, preferably by heat sealing. The units may be separably connected and wound into a roll on the packaging apparatus.

FIG. 4

EP 0 124 732 A2

PACKAGING SYSTEM FOR AN OCCLUSIVE DRESSING

BACKGROUND OF THE INVENTION

This invention relates to a packaging system for dressings used in surgical and general medical applications such as bandaging and the like.

Various types of surgical dressings are well known in the art. Exemplary of such dressings are those disclosed in U.S. Patent Nos. 3,645,835; 3,121,021; and 4,255,550 as well as another Patent Application in USA filed in 1983 by George F. Feeley and A. Frederick Karakas entitled "Occlusive Dressing and Delivery System Therefor", the disclosure of which is incorporated herein by reference and made a part hereof.

Generally, the basic constructs of occlusive dressings are primarily the same. This construct involves a reinforced or unreinforced polymeric sheet material, a pressure-sensitive adhesive affixed thereto, a release liner and, optionally, an absorbent material such as a cotton pad or the like. In use, the release liner is removed and the adhesive is contacted to the patient's skin with the polymeric sheet material acting as the occlusive barrier.

Workers in the field have modified the polymeric sheet material and the adhesive to provide properties which are intended to have the occlusive dressing approximate the properties of human skin. For example, it is desired that the occlusive dressing, i.e. the combination of the polymeric sheet material and the adhesive, have a moisture vapor permeability of greater than 300 $g/m^2/24$ hrs. at $40^\circ C$ and 80 percent relative humidity. Although there have been great strides in providing occlusive dressings with the desired physicochemical properties, there have been certain problems which have arisen in the delivery of these occlusive dressings to the patient. In many instances, moisture vapor permeability is a function of the film thickness of a polymeric material, and the thinner the material the greater the moisture vapor permeability. Typically, the polymeric material is 1 to 5 mils and the commercial experience has been on the order of 1 mil or greater.

Typically, occlusive dressings are manufactured in bulk form with the pressure-sensitive adhesive being applied to the polymeric film along with a release liner affixed to the surface of the pressure-sensitive adhesive as previously described. There are several processes for the manufacture of such occlusive dressings, but predominant in the field are primarily well recognized sheet goods coating techniques which employ large rolls of material, i.e. three to six feet wide and hundreds of feet long. After the material has been formed in bulk, it is unwound from the roll and cut into the desired configuration for use in particular applications well recognized for occlusive dressings, such as in strips; large sheets, i.e. one foot by one foot or greater; and the like. After cutting into the desired configuration, the dressings must be packaged and sterilized. Typically, the individual dressings are placed in bacteria proof containers of sheet goods and sealed along the edges. The packaging is done by hand or machine and constitutes a separate step in the manufacturing process. In use, the packaging is stripped away and discarded and the dressing is then used.

Workers in the art have desired to simplify the packaging process and efforts have resulted primarily in manufacturing and assembly improvements packaging, rather than a change in the dressings themselves. In accordance with the present invention, a packaging system is provided wherein the medical dressing is prepared and packaged in a single process, thereby eliminating processing and labor costs. In addition, in accordance with the invention, necessary portions of the medical dressing are utilized as portions of the packaging, thereby eliminating material costs.

## SUMMARY OF THE INVENTION

According to the invention occlusive dressings are continuously packaged from dressing constructs comprising of a first carrier sheet, a film of polymeric material adhered to a surface of said first carrier sheet, an adhesive material affixed to the opposed surface of said first carrier sheet and a release liner adhered to the surface of said adhesive opposed to the film of polymeric material. Sealed dressing units are packaged by means which continuously transports the constructs to a cutting means where a continuous composite comprising the release liner, adhesive and polymeric film (but not the first carrier sheet) are cut through to define a plurality of individual dressing constructs with a continuous first carrier sheet. The excess of the cut composite is then removed by take up means to expose the margin of the first carrier sheet and to provide discrete dressing composites along the first carrier sheet. Next, a second carrier sheet is superimposed on the surface of said composites opposed to said first carrier sheet and the carrier sheets are sealed to each other, preferably by heat sealing, along the margin of each composite to provide a dressing unit which is completely sealed in an individual package. The packages may be separably connected, i.e. perforated in the areas where they are connected, and wound into a roll on an exit roll of the packaging apparatus. The second carrier sheet may be pre-printed or printed after it is sealed to the first carrier sheet.

In forming the occlusive dressing, the carrier sheet is mated with the film of polymeric material. The film of polymeric material may be an extruded film in which case the film is simply mated with the carrier. In the most preferred embodiment in forming the occlusive dressing, an aqueous polyurethane is cast onto the carrier sheet and the water is removed therefrom by drying or the like.

The aggressively tacky pressure sensitive adhesive in a solvent or aqueous medium is cast onto the release liner and dried.

Thus, a film of polymeric material is applied to the carrier sheet and release liner is coated with adhesive. The free surface of the adhesive is then brought in contact with the free surface of the film of polymeric material and the two surfaces are laminated together.

The carrier sheet useful in the practice of the invention is a film formed of a polymeric material having a thickness of about 1 to 5 mils. Preferably, the carrier sheet is made of a highly flexible polymeric material having sufficient strength or modulus so as not to have a tendency to sag and fold onto itself during the formation of the occlusive dressing or during delivery of the occlusive dressing. It is to be noted that the carrier sheet should be highly drapable and flexible and have a tear strength of greater than that of the film and a modulus of 500 psi or greater. In this regard, it is preferred that the carrier sheet be an amorphous polymer such as a polyolefin, i.e. polyethylene or polypropylene, as opposed to an oriented high modulus polymer such as polyethylene terephthalate. The oriented polymers are less deformable than the amorphous polymers, and thus, conformance to large areas of the body of the patient is more difficult with the high modulus oriented polymeric carrier sheets. Most preferably, the carrier sheet is transparent or translucent so that the person applying the occlusive dressing to the patient can readily observe the patient therethrough and properly position the occlusive dressing on the patient.

The film of polymeric material is preferably of a film thickness of 0.25 to 5 mils, and typically has a tensile strength of about 1,500 to about 8,000 psi and a modulus of 500 to 3,000 psi at the given film thickness. It is to be noted that low modulus, low tensile strength materials are useful in forming the film of the occlusive dressing because of the unique construct in accordance with the invention. The polymeric material useful in the practice of the invention may be a polyacrylate, polyamide, polyolefin, polyvinyl halide or the like. However, it is preferred that thermoplastic polyurethane polymers be used as the synthetic polymeric material since these polymers have the wide range of tensile and elongation properties required for the final product. Preferably, a low modulus polymer, i.e. a modulus of about 500 to 3,000 psi at the specified film thickness, is particularly useful in the practice of the invention since the low modulus polymeric materials, in film form, readily conform to the body.

## DESCRIPTION OF THE DRAWINGS

Fig. 1 is a longitudinal view of an occlusive dressing construct useful in the invention;

Fig. 2 is a schematic view of apparatus for continuously packaging dressings from the construct of Fig. 1 according to the invention;

Fig. 3 is a top plan view of preferred apparatus for packaging occlusive dressings;

Fig. 4 is a sectional view of the apparatus taken along lines IV-IV of Fig. 3; and

Fig. 5 is a section view of a complete sealed occulsive dressing unit.

## BRIEF DESCRIPTION OF THE INVENTION

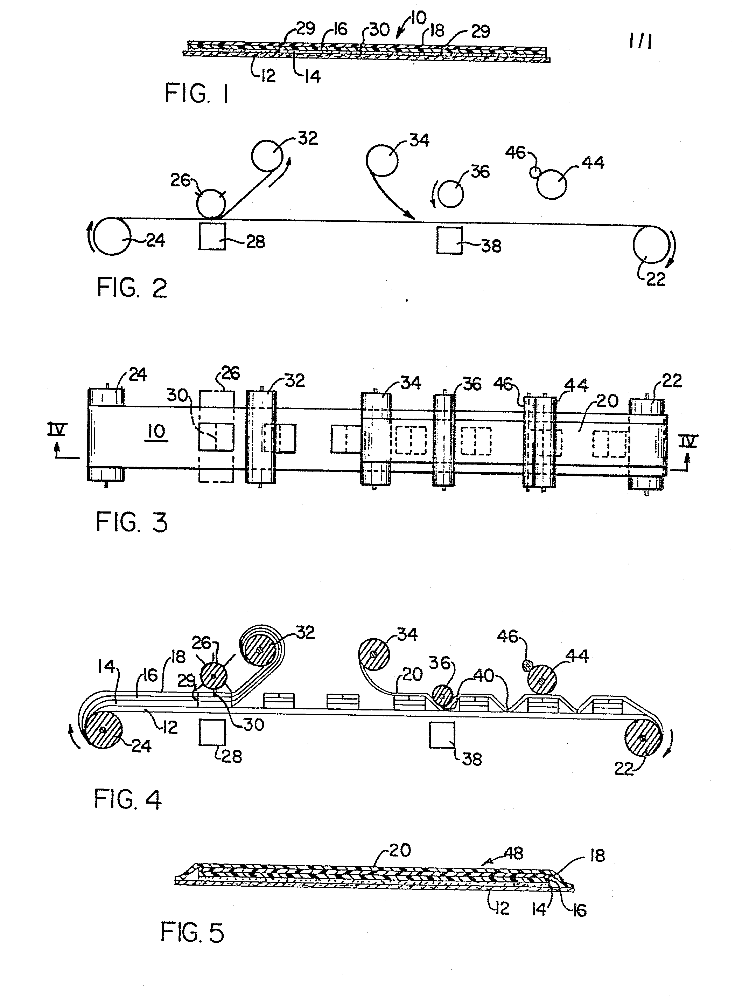

A basic dressing construct 10 to be packaged according to the invention is shown in Fig. 1 and comprises a first carrier sheet 12, a film of polymeric material 14, an adhesive 16, and a release liner 18. The formation of the construct 10 is fully described in U.S. Patent Application as mentioned above . Preferably, the polymeric material is moisture vapor permeable polyurethane.

The dressing construct 10 may be formed in a continuous process or taken up in a roll. For the purpose of describing the present invention, it is assumed that the construct is in the form of a roll; however, it should be understood that the process is equally suitable for packaging dressings directly from the apparatus which forms the constructs.

A construct is continuously transported, i.e. unrolled by driving or take up roll 22 from driven or supply roll 24 and is passed under cutting means such as a rotary die cutter 26 with backing stand 28 which slits (as at 29 in Fig. 1) through release liner 18, adhesive 16 and polymeric material 14 forming a composite on the carrier sheet 12. The die cutter 26 may also provide a score 30 in the release layer 18 which facilitates removal of the release layer during application of the dressing. The excess or waste composite material separated by the die cutter is removed on take up reel 32 and discarded as often as necessary.

A second carrier sheet 20 provided on roll 34 is unwound and superimposed over release liner 18 with proper tension being maintained on the carrier sheet 20. A suitable material for forming the second carrier sheet 20 is any material which will form a seal with the first carrier sheet 12. The material should be impervious to moisture and bacteria and should withstand sterilization. Preferably, the material should be flexible enough to enable the packaged unit to be wound into a roll, particularly in a continuous process such as that described.

The second carrier sheet 20 may be precoated so that it adheres by heat sealing or the like to the margin of the first carrier sheet 12 as the second carrier sheet 20 is taken off roll 34 and onto the first carrier sheet. In a preferred embodiment the carrier sheets are sealed along their margins by a heat sealer 36 with backing stand 38 which also may be used to form perforations 40 at the juncture of the carrier sheets 12 and 20 to permit the packaged units to be easily separated from one another for use. The sealing and perforating may be performed by separate units if desired.

Prior to the packaged units being wound on take up roll 22, the second carrier sheet 20 on each unit may be printed by rotary printer 44 which carries ink roller 46. Of course, the carrier sheet could be printed before it is applied to the unit or it could be preprinted, thus eliminating the need for a separate printer.

The completely sealed dressing units 48 are shown in Fig. 5 and include first carrier sheet 12, polymeric material 14, adhesive 16, release liner 18 and second carrier sheet 20. The units may be separated from the roll by hand for individual use or the entire roll of packaged dressing units 48 can be sterilized and placed in an examining or operating room. Each unit can be separated as needed from the roll along perforations 40 and opened without contaminating the remaining units on the roll.

The invention provides an occlusive dressing packaging system which results in reduced packaging costs.

Having described a presently preferred embodiment of my invention, it is to be understood that it may otherwise be embodied within the scope of the appended claims.

THE CLAIMS:

1.    A method for packaging occlusive dressings comprising the steps of:

(a)    transporting a continuous dressing construct comprising a first carrier sheet, a film of polymeric material adhered to one surface of said first carrier sheet, an adhesive material affixed to the opposed surface of said first carrier sheet and a release liner adhered to the surface of said adhesive opposed to the film of polymeric material to a cutting means;

(b)    cutting through the composite comprising the release liner, adhesive and polymeric film but not through the first carrier sheet to define a plurality of individual dressing constructs with a continuous first carrier sheet;

(c)    removing the continuous sheet of cut composite from the first carrier sheet to expose the margins of the first carrier sheet and to provide discrete dressing composites along said first carrier sheet;

(d)    superimposing a second carrier sheet on the surface of said composites opposed to said first carrier sheet; and

(e)    sealing said carrier sheets to each other along the margin of each composite to provide a dressing unit wherein each dressing is completely sealed in an individual package.

2.    The method of claim 1 wherein said first carrier sheet is a continuous sheet and dressing units formed thereof are separately connected and wound into a roll.

3.    The method of claim 2 and perforating said first and second carrier sheets between said units where said sheets are joined to define individual package units.

4.    The method of claim 1 wherein said first and second carrier sheets are sealed by heat sealing.

5.    The method of claim 1 and scoring said release liner to facilitate separation of the liner from the film when the dressing is used.

6.    A package for an occlusive dressing comprising:

(a)    a first carrier sheet;

(b)    a film of polymeric material adhering to one surface of said first carrier sheet, the margin of said first carrier sheet extending beyond the margin of the film of polymeric material;

(c)    an adhesive material affixed to the surface of said polymeric material opposing the surface of said first carrier sheet;

(d)    a release liner adhered to the surface of said adhesive opposing said polymeric material; and

(e)    a second carrier sheet superimposed on the surface of said release liner opposing said adhesive and sealed to said first carrier sheet so that said polymeric material, adhesive material and release liner are completely sealed between said carrier sheets.

7.    A package for an occlusive dressing as set forth in claim 6 wherein said polymeric material is a moisture vapor permeable polyurethane.

8.    A package for an occlusive dressing as set forth in claim 6 wherein said second carrier sheet is flexible.

9.    A package for an occlusive dressing as set forth in claim 6 wherein said first and second carrier sheets are heat sealed to each other.

10.    Apparatus for continuously packaging occlusive dressings comprising:

(a)    entry means for transporting dressing constructs including a continuous first carrier sheet;

(b)    means for cutting the constructs into discrete components on said first carrier sheet;

(c)     means for removing excess composite from the first carrier sheet to expose the margins of said first carrier sheet and to provide discrete dressing composites along said first carrier sheet;

(d)     means for superimposing a second carrier sheet on the surface of said composites opposed to said first carrier sheet;

(e)     means for sealing said first and second carrier sheets to each other along the margin of said composite to provide a dressing unit wherein each dressing is completely sealed in an individual package; and

(f)     exit means for taking up the packages as they are continuously formed.

11.    Apparatus as set forth in claim 10 and including means for printing said second carrier sheet.

12.    Apparatus as set forth in claim 10 and including means for perforating said first and second carrier sheets in the area where they are sealed to one another.

13.    Apparatus as set forth in claim 10 and including means for scoring the release liner of said construct.

14.    Apparatus as set forth in claim 10 wherein said sealing means comprises a heat sealer.

15.    Apparatus as set forth in claim 10 wherein at least one of said entry means and said exit means is a roll.

0124732

1/1

FIG. 1

FIG. 2

FIG. 3

FIG. 4

FIG. 5